# EUROPÄISCHE PATENTSCHRIFT

(11) **EP 3 019 126 B1**
(45) Veröffentlichungstag und Bekanntmachung des Hinweises auf die Patenterteilung: **06.09.2017**
(21) Anmeldenummer: 14738391.3
(22) Anmeldetag: 08.07.2014
(51) Int. Cl.: A61F 5/01

(54) **ORTHESENGELENK**
ORTHOSIS JOINT
ARTICULATION POUR ORTHÈSE

(30) Priorität: 09.07.2013 DE 102013011382
(43) Veröffentlichungstag der Anmeldung: 18.05.2016
(73) Patentinhaber: Otto Bock Healthcare GmbH, 37115 Duderstadt (DE)
(72) Erfinder: LÜRSSEN, Marcus, 37073 Göttingen (DE); HOCHMANN, David, 48565 Steinfurt (DE); AUBERGER, Roland, A-1140 Wien (AT); SIEWERT, Gordon, 37083 Göttingen (DE); BREUER-RUESCH, Christian, A-1230 Wien (AT)
(74) Vertreter: Stornebel, Kai
(86) Internationale Anmeldenummer: PCT/EP2014/001877
(87) Internationale Veröffentlichungsnummer: WO 2015/003802

(56) Entgegenhaltungen:
- DE-A1-102010 014 334
- US-A- 5 372 574
- US-A- 5 899 869
- US-A- 6 080 123
- US-B2- 6 969 365

## Beschreibung

Die Erfindung betrifft ein Orthesengelenk mit einem Grundkörper, der eine Aufnahme für eine proximale Komponente sowie eine Lagerstelle für eine schwenkbar an dem Grundkörper angeordnete distale Komponente und auf die distale Komponente einwirkende Anschläge oder Federelemente aufweist. Ein solches Orthesengelenk ist insbesondere an Orthesen unterer Extremitäten wie eine Knöchel-Fuß-Orthese (AFO) oder eine Knie-Knöchel-Fuß-Orthese (KAFO) vorteilhaft einsetzbar, ist jedoch nicht auf diesen Einsatzzweck beschränkt.

Orthesen dienen zur Unterstützung der Bewegung natürlicher Gliedmaßen oder zu deren Stabilisierung. Dazu sind in der Regel Stützstrukturen in Gestalt von Schalen oder Schienen vorgesehen, die an Gliedmaßen befestigt werden. Sofern es sich nicht um Immobilisierungsorthesen handelt, sind im Bereich der natürlichen Gelenke zwischen den Stützeinrichtungen Gelenke vorgesehen, die eine Beweglichkeit zueinander zulassen. Die Bewegung kann in ihrer Richtung oder in ihrem Umfang eingeschränkt werden, sofern dies aus therapeutischer Sicht sinnvoll ist. Darüber hinaus kann der Bewegung ein Widerstand entgegengesetzt werden oder Rückstellkräfte so aufgebracht werden, dass eine Rückkehr in eine Ausgangsstellung erfolgt oder zumindest erleichtert wird.

Die DE 10 2010 014 334 A1 betrifft ein Orthesengelenk mit zwei Funktionsmitteln zum Bilden zweier Anschläge. Die Funktionsmittel sind als Tellerfederanordnung ausgebildet, die in Federkanälen angeordnet sind. Die Federkanäle sind in dem Grundkörper ausgebildet und wirken auf einen schwenkbar an dem Grundkörper angeordneten Bügel. Die Vorspannung der Tellerfederanordnung kann über ein Stellelement, das in den Federkanal eingeschraubt wird, verändert werden.

Die US-A-4,489,718 betrifft ein Knieorthesengelenk, über das der maximal erreichbare Kniewinkel eingestellt werden kann. In einem Grundkörper ist ein durchgehender Kanal angeordnet, in dem zwei Federn angeordnet sind, die auf einem Zapfen eines gelenkig an dem Grundkörper gelagerten Schwenkteils eingreifen. Über eine Änderung der Vorspannung der Federn durch zwei einschraubbare Stellelemente ist es möglich, den maximal erreichbaren Kniewinkel sowohl in Flexionsrichtung als auch in Extensionsrichtung einzustellen.

Die US-A-4,771,768 betrifft eine Knöchelorthese zur Versorgung von Knöchelbrüchen. Ein Fußteil ist gelenkig an einer Unterschenkelschiene befestigt. An der Unterschenkelschiene ist ein Grundkörper angeordnet, in dem zwei Metallstifte eingeführt sind, die über Einstellschrauben in Richtung auf Vorsprünge am Fußteil bewegt oder entfernt werden können. Über diese Metallstifte kann der Bewegungsumfang und der maximal erreichbare Knöchelwinkel eingestellt werden.

Problematisch an den aus dem Stand der Technik bekannten Prothesengelenken ist die Tatsache, dass sie in ihrer Ausgestaltung auf den jeweiligen Einsatzzweck abgestimmt sind. Eine Individualisierung und Anpassung des Orthesengelenkes an den jeweiligen Nutzer und gegebenenfalls an einen Therapiefortschritt ist nicht oder nur mit sehr hohem Aufwand möglich.

Die US 6,080,123 A betrifft ein Orthesengelenk mit einer an einem Grundkörper angeordneten Hydraulikeinheit, über die mehrere Kugeln gegen ein schwenkbar an dem Grundkörper gelagertes Unterteil mit einer Kraft beaufschlagt wird. In einem einschraubbaren Rohr mit einem externen Hydraulikanschluss ist ein beweglicher Kolben angeordnet, der auf Lagerkugelkörper drückt, die wiederum auf das Unterteil einwirken.

Die US 6,969,365 B2 betrifft eine einstellbare Orthese mit einem Stützelement, das zwei über ein Gelenk miteinander verbundene Schenkel aufweist. Die Orthese ist über Befestigungsmittel an einer Gliedmaße festlegbar. Ein Lastwandler ändert zumindest einen Schenkel oder ein Befestigungselement als Reaktion auf ein erstes Signal.

Aufgabe der vorliegenden Erfindung ist es, ein Orthesengelenk bereitzustellen, das leichter individualisierbar ist.

Erfindungsgemäß wird diese Aufgabe durch ein Orthesengelenk mit den Merkmalen des Hauptanspruches gelöst. Vorteilhafte Ausgestaltungen und Weiterbildungen der Erfindung sind in den Unteransprüchen, der Beschreibung sowie den Figuren aufgeführt.

Das erfindungsgemäße Orthesengelenk mit einem Grundkörper, der eine Aufnahme für eine proximale Komponente sowie eine Lagerstelle für eine schwenkbar an dem Grundkörper angeordnete distale Komponente und auf die distale Komponente einwirkende Anschläge oder Federelemente aufweist, sieht vor, dass an dem Grundkörper zumindest ein Aufnahmeelement lösbar befestigt ist, in oder an dem ein mit der distalen Komponente wechselwirkendes Funktionselement angeordnet ist. Während bei Orthesengelenken aus dem Stand der Technik die darin angeordneten Anschläge oder Federelemente vollständig in dem Grundkörper aufgenommen und darin geführt sind, ist erfindungsgemäß vorgesehen, dass ein Aufnahmeelement für ein Funktionselement lösbar an dem Grundkörper befestigt ist, so dass das Aufnahmeelement austauschbar und an die individuellen Anforderungen des Nutzers anpassbar ist. Dadurch ist es möglich, unterschiedliche Funktionselemente oder aber auch unterschiedliche Ausgestaltungen eines Funktionselementes, beispielsweise einer Feder, in dem Aufnahmeelement aufzunehmen oder zu führen, so dass eine Anpassung an die jeweiligen Erfordernisse leicht erfolgen kann. Das Aufnahmeelement steht über die Kontur des Grundkörpers hinaus, so dass bei einem nicht an dem Grundkörper befestigten Aufnahmeelement ein schlankes Design des Grundkörpers und damit der gesamten Orthese erreicht werden kann. Die Aufnahmeelemente und die Funktionselemente sind als integrierte Baugruppe ausgebildet, so dass ein modularer Aufbau des Orthesengelenkes einfach verwirklicht werden kann. In den jeweiligen Aufnahmeelementen sind Funktionselemente integriert oder vorgefertigt eingebaut, so dass eine Anpassung und Individualisierung des Orthesengelenkes einfach durch Austausch der funktionalisierten Aufnahmeelemente erfolgen kann.

Das Aufnahmeelement ist vorteilhafterweise formschlüssig reversibel an dem Grundkörper festgelegt, beispielsweise über ein Gewinde, das korrespondierend an dem Aufnahmeelement und dem Grundkörper vorgesehen ist. Ebenfalls ist es möglich, das Aufnahmeelement über einen Bajonettverschluss oder Klammern, federnde Rastlaschen oder andere Formschlusselemente an dem Grundkörper festzulegen.

Das Aufnahmeelement kann als eine Hülse mit einem Gewinde ausgebildet sein, um einerseits das Aufnahmeelement an dem Grundkörper festzulegen oder andererseits das Funktionselement innerhalb des Aufnahmeelementes zu positionieren und einzustellen.

Das Funktionselement kann als Feder, Federpaket, Dämpfer, Sensor oder als Anschlag oder einer Kombination daraus ausgebildet sein. Das jeweilige Aufnahmeelement ist in Form und Ausstattung auf das jeweilige Funktionselement abgestimmt, werden Dämpfer, Federn oder Federpakete verwendet, dient das Aufnahmeelement als Außenführung für den Dämpfer, die Feder oder das Federpaket. Werden Sensoren als Funktionselemente eingesetzt, ist das Sensorgehäuse das Aufnahmeelement und weist gegebenenfalls Schnittstellen zur Weiterleitung von Sensordaten sowie Festlegungseinrichtungen zum Befestigen an dem Grundkörper auf. Anschläge, die den Bewegungsumfang in dem Gelenk definieren, können ebenfalls in dem Aufnahmeelement angeordnet sein. Zur verstellbaren Positionierung der jeweiligen Anschläge ist es vorgesehen, dass Einstelleinrichtungen an dem Aufnahmeelement vorgesehen sind, um eine Anpassung vornehmen zu können, ohne den Anschlag selbst austauschen zu müssen.

Es können mehrere Funktionselemente seriell hintereinander angeordnet sein, um unterschiedliche Funktionen miteinander zu kombinieren. Beispielsweise können eine Feder und ein Sensor seriell angeordnet sein, um als Modul Informationen über die aufgebrachte Kraft oder den Verlagerungsweg zu liefern, ebenso können serielle Feder-Dämpfer-Kombinationen eingesetzt oder Federn unterschiedlicher Steifigkeiten zur Erreichung einer progressiven Kennlinie hintereinander angeordnet werden.

Das Funktionselement kann als induktiver Sensor ausgebildet ist, der einen Verlagerungsweg erfasst, der beispielsweise durch Federn, Federpakete oder Dämpfer ermöglicht wird, so dass der Verlagerungsweg und/oder eine Winkelstellung über den induktiv arbeitenden Sensor ermittelt werden kann.

Mehrere Aufnahmeelemente können seriell hintereinander angeordnet sein, beispielsweise durch eine Verschraubung oder andere Formschlusselemente wie Bajonettverschluss oder Rastverbindungen. Dazu sind an den Aufnahmeelementen, die miteinander gekoppelt werden sollen, korrespondierende Verschraubungen oder Formschlusselemente vorgesehen, so dass ein Außengewinde in ein Innengewinde eingeschraubt oder die korrespondierenden Formschlusselemente miteinander verriegelt werden kann oder können, um entweder eine Verlängerung des Aufnahmeelementes für ein entsprechend großes Funktionselement oder eine Kombination unterschiedlicher Funktionselemente miteinander zu ermöglichen. Statt beispielsweise mehrerer Aufnahmeelemente in Gestalt von Kanälen mit der jeweils richtigen Länge vorrätig halten zu müssen, können mehrere kurze Aufnahmeelemente miteinander zu einem Aufnahmeelement oder einem Kanal der gewünschten Länge kombiniert werden.

Das Aufnahmeelement ist vorteilhafterweise in einer Ausnehmung in dem Grundkörper eingesetzt, in dem Formschlusselemente zur Befestigung des Aufnahmeelementes angeordnet sind. Die Formschlusselemente können als Gewinde, Führungsbahn für Bajonettverschlüsse, Federzungen, Vorsprünge oder dergleichen ausgebildet sein. Von der Ausnehmung führt ein Kanal in Richtung auf die distale Komponente, um eine Wirkverbindung mit der distalen Komponente, beispielsweise einem Absatz eines Gelenkteils, herzustellen. Stifte, Bolzen oder andere Druckelemente können durch den Kanal hindurchgeführt werden, um Kräfte von der distalen Komponente auf das Funktionselement zu leiten. Ebenfalls ist es möglich, dass das Funktionselement unmittelbar auf die distale Komponente durch den Kanal hindurch einwirkt, beispielsweise indem Federn direkt auf Auflageflächen der distalen Komponente aufliegen oder ein Anschlag bei Erreichen der maximalen Winkelstellung auf einem Auflager auftrifft. Auch ist es möglich, dass durch den Kanal ein Sensor die Position der distalen Komponente relativ zum Grundkörper erfasst oder die auf die distale Komponente einwirkenden Kräfte aufnimmt. Dadurch ist es beispielsweise möglich, das Gelenk im Rahmen einer gesteuerten Orthese einzusetzen und über die Sensoren gewünschte Informationen über den Bewegungszustand, die Art der Bewegung und dergleichen zu erhalten.

Die Aufnahme an dem Grundkörper kann als Schienenkasten ausgebildet sind, in dem eine Orthesenschiene, beispielsweise für ein Unterschenkelteil oder ein Oberschenkelteil, festgelegt wird. Die Festlegung kann klemmend und/oder formschlüssig erfolgen, beispielsweise über eine doppelte Schraubverbindung und konisch zulaufenden Seitenwände an den Schienen und/oder dem Schienenkasten.

Die distale Komponente kann als Schiene, Schienenkasten, Schale, Fußbügel oder Fußstütze ausgebildet sein. Unter einer Fußstütze werden Komponenten verstanden, die den Fuß untergreifen, Fußbügel sind Komponenten, die sich auch parallel zu dem Fuß erstrecken können, Fußbügel können in einer Fußstütze enden.

Die Aufnahmeelemente sind vorteilhafterweise beidseitig der Lagerstelle angeordnet, so dass die Funktionselemente sowohl hinsichtlich einer Flexion als auch einer Extension wirksam angeordnet werden können.

Nachfolgend werden Ausführungsbeispiele der Erfindung anhand der beigefügten Figuren näher erläutert. Es zeigen:
- Figuren 1 - 5 -: verschiedene Ansichten eines Grundkörpers ohne Aufnahmeelemente;
- Figur 6 -: eine Variante der Figur 1 mit einem eingeschraubten Anschlagkanal;
- Figur 7 -: eine Schnittdarstellung der Figur 6;
- Figur 8 -: eine Explosionsdarstellung der Figur 6;
- Figur 9 -: Figur 8 in Schnittdarstellung;
- Figur 10 -: eine Schnittdarstellung gemäß Figur 7 mit beidseitig eingeschraubten Aufnahmeelementen;
- Figur 11 -: eine Explosionsdarstellung der Figur 10;
- Figur 12 -: eine Variante der Figur 10 mit eingeschraubten Federaufnahmen;
- Figur 13 -: eine Schnittdarstellung der Figur 12;
- Figur 14 -: eine perspektivische Darstellung eines Grundkörpers mit eingeschraubtem Sensor;
- Figuren 15 bis 21 -: Draufsichten und Schnittdarstellungen von Varianten eines Orthesengelenkes.

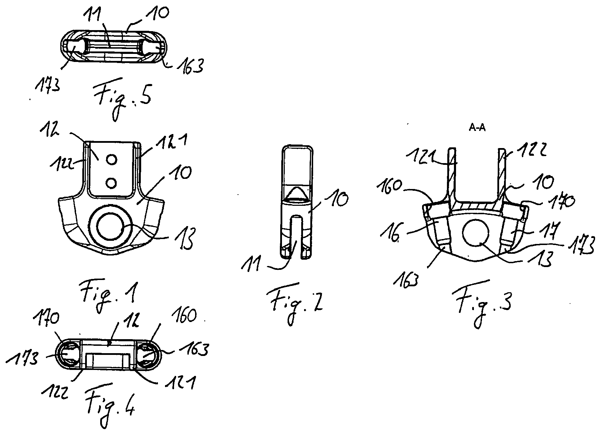

Figur 1 zeigt in einer Draufsicht einen Grundkörper 10 eines Orthesengelenkes mit einer Aufnahme 12 für eine nicht dargestellte proximale Komponente, beispielsweise eine Orthesenschiene, sowie eine Lagerstelle 13 zur Aufnahme einer Schwenkachse, an der eine nicht dargestellte, distale Komponente schwenkbar befestigbar ist.

Figur 2 zeigt den Grundkörper 10 in einer nach rechts geklappten Darstellung. Es ist zu erkennen, dass in dem Grundkörper 10 ein Schlitz 11 ausgebildet ist, der zur Aufnahme der distalen Komponente dient. Die beiderseits des Schlitzes 11 angeordneten Stege des Grundkörpers 10 weisen jeweils die Durchgangsbohrung für die Schwenkachse auf und bilden die Lagerstelle 13 für die distale Komponente.

Figur 3 zeigt den Grundkörper 10 in einer Schnittdarstellung mit der Lagestelle 13 und den beiden senkrecht nach oben zeigenden Armen der seitlichen Wände 121, 122 der Aufnahme 12. Die Aufnahme 12 ist als wannenförmiger Schienenkasten ausgebildet und weist an der weggeschnittenen Grundwand Ausnehmungen auf, durch die Formschlusselemente, beispielsweise Schrauben, hindurchgeführt werden können, um eine Orthesenschiene an dem Grundkörper 10 zu befestigen.

Beiderseits der Lagerstelle 13 sind Ausnehmungen 16, 17 in Gestalt von Bohrungen vorhanden, die an dem proximalen Ende Formschlusselemente 160, 170 aufweisen, die als Innengewinde oder Rastelemente für einen Bajonettverschluss ausgebildet sein können. Alternativ dazu können die Formschlusselemente 160, 170 als federnd gelagerte Rasteinheiten ausgebildet sein. An dem den Formschlusselementen 160, 170 gegenüberliegenden Enden der Ausnehmungen 16, 17 sind Kanäle 163, 173 vorgesehen, die in distale Richtung weisen, so dass ein freier Durchgang zu der nicht dargestellten distalen Komponente möglich ist.

Die freien Durchgänge beiderseits der Lagerstelle 13 sind als Kanäle 163, 173 in den Figuren 4 und 5 zu erkennen. In der Figur 4 ist zudem die kastenförmige Ausgestaltung der Aufnahme 12 mit der Grundwand und den beiden nach vorne ragenden Wänden 121, 122 zu erkennen. Figur 4 zeigt ebenfalls die Formschlusselemente 160, 170 in Gestalt von Gewinden, Figur 5 zeigt die durchgängigen Kanäle 163, 173 ebenso wie den Schlitz 11 an dem distalen Abschnitt des Grundkörpers 10.

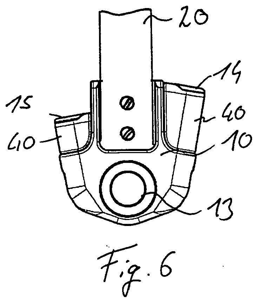

In der Figur 6 ist der Gelenkgrundkörper 10 mit einer montierten proximalen Komponente 20 in Gestalt einer Orthesenschiene dargestellt. Die Orthesenschiene 20 ist über Schrauben an dem Grundkörper 10 festgelegt. In der Darstellung der Figur 6 sind zwei Aufnahmeelemente 14, 15 beiderseits der proximalen Komponente 20 an dem Grundkörper 10 befestigt. Um die Aufnahmeelemente 14, 15 herum sind Verkleidungsteile 40 angeordnet, um einen möglichst glattwandigen und kompakten Aufbau des Orthesengelenkes zu ermöglichen.

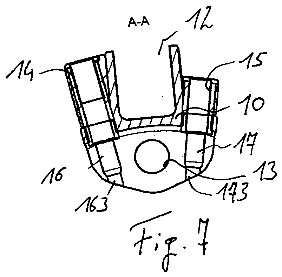

In der Figur 7 ist in einer gedrehten, geschnittenen Darstellung der Grundkörper 10 gemäß Figur 6 dargestellt. Auch hier sind die beiden symmetrisch beidseitig zur Lagerstelle 13 angeordneten Ausnehmungen 16, 17 zu erkennen, die in den Kanälen 163, 173 münden. Die Aufnahmeelemente 14, 15 sind in die Gewinde 160, 170, wie sie in der Figur 3 dargestellt sind, eingeschraubt. Durch das Einschrauben der Aufnahmeelemente 14, 15 werden auch die beiden Verkleidungen 40 an dem Grundkörper fixiert.

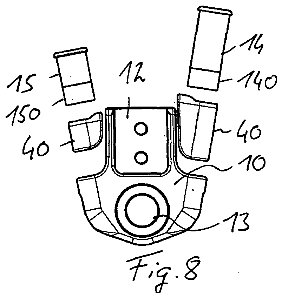

Figur 8 zeigt die Variante der Figur 6 in einer Explosionsdarstellung. Es ist zu erkennen, dass die Verkleidungen 40 eine zu der Kontur des Grundkörpers 10 komplementäre Formgebung aufweisen, so dass die Verkleidungen 40 die äußere Kontur ergänzen. Aufgrund des klappsymmetrischen Aufbaus ist es möglich, die beiden unterschiedlich langen Aufnahmeelemente 14, 15 entweder in der linken oder in der rechten Ausnehmung 16, 17 festzulegen. Zum Festlegen der Aufnahmeelemente 14, 15 werden die als Hülsen ausgebildeten Aufnahmeelemente in die Gewinde 160, 170 eingeschraubt. Dazu sind an den distalen Enden der Aufnahmeelemente 14, 15 Außengewinde 140, 150 angeordnet. An den proximalen Enden sind Absätze angeordnet, die so ausgebildet sind, dass sie auf den oberen Rändern der Verkleidungen 40 aufliegen, so dass zusammen mit dem Einschrauben der Aufnahmeelemente 14, 15 eine Festlegung der Verkleidungen 40 an dem Grundkörper 10 erfolgt.

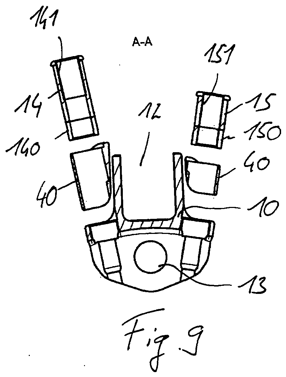

Figur 9 zeigt die Darstellung der Figur 8 in Schnittdarstellung. Es ist zu erkennen, dass die hülsenförmigen Aufnahmeelemente 14, 15 an dem distalen Ende Außengewinde 140, 150 aufweisen. Darüber hinaus sind die Aufnahmeelemente 14, 15 mit Innengewinden 141, 151 versehen, um in den Aufnahmeelementen 14, 15 angeordnete Funktionselemente, die nicht dargestellt sind, darin zu fixieren und gegebenenfalls in Richtung auf die distale Komponente vorzuspannen. Die Verkleidungen 40 weisen Durchgangsöffnungen auf, ebenso wie die Aufnahmeelemente 14, 15, so dass eine durchgängige, unmittelbare Verbindung von dem oberen Rand der Aufnahmeelemente 14, 15 bis zur Öffnung der Kanäle 163, 173 vorhanden ist. Aufgrund der symmetrischen Ausgestaltung sowohl des Grundkörpers 12 als auch der Verkleidungen 40, ist es möglich, die jeweiligen Aufnahmeelemente 14, 15 an einer der beiden Ausnehmungen 16, 17 zu befestigen und das Orthesengelenk sowohl links als auch rechts einzusetzen. Im dargestellten Ausführungsbeispiel ist das kürzere Aufnahmeelement 15 zur Aufnahme eines Anschlages vorgesehen, während das längere Aufnahmeelement 14 zur Aufnahme eines Federpaketes vorgesehen ist.

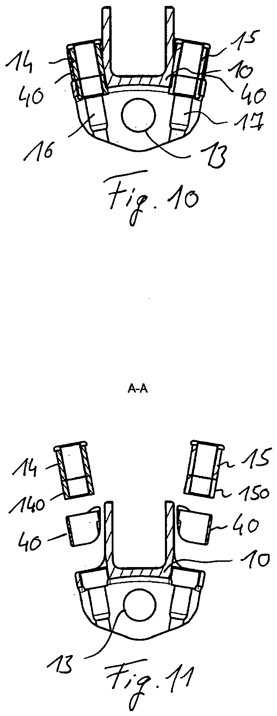

In den Figuren 10 und 11 sind Varianten der Figuren 7 und 9 dargestellt, bei der statt eines langen, als Federkanal dienenden Aufnahmeelementes 14 zwei gleichlange Aufnahmeelemente 14, 15 in Hülsenform in den Grundkörper 10 eingeschraubt sind. Beide Aufnahmeelemente 14, 15 dienen vorliegend als ein Anschlagkanal.

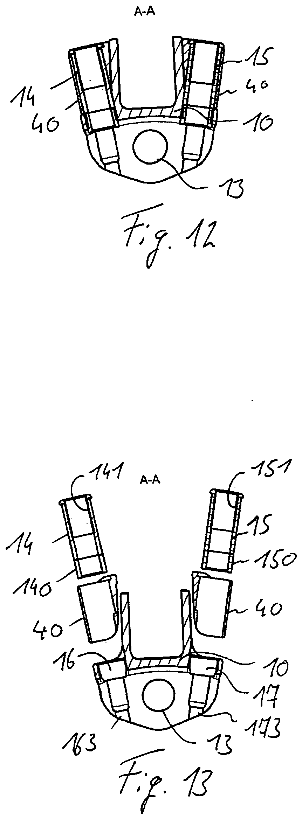

Die Figuren 12 und 13 zeigen eine Variante der Erfindung, bei der statt zweier eingeschraubter Anschläge, wie in den Figuren 10 und 11 dargestellt, beidseitig neben der Lagerstelle 13 in den Ausnehmungen 16, 17 zwei Federkanäle als Aufnahmeelemente 14, 15 eingeschraubt sind.

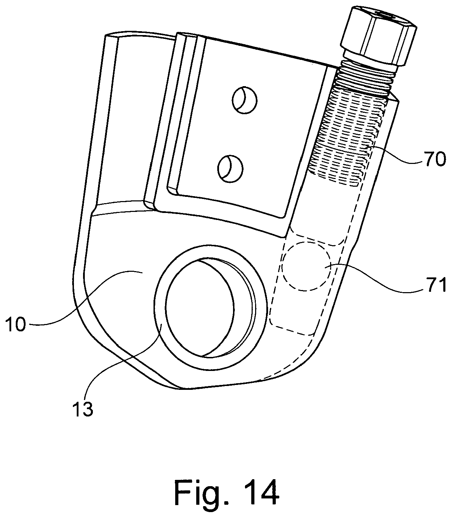

In der Figur 14 ist eine weitere Variante der Erfindung dargestellt, bei der in dem Grundkörper 10 in einer Ausnehmung 16 ein Sensor 70 als Funktionselement eingeschraubt ist. Das Sensorgehäuse dient dabei als Aufnahmeelement und weist ein Außengewinde auf, das in das Innengewinde 160 der Aufnahme 16 eingeschraubt ist. An dem distalen Ende des Sensors 70 ist als Kontaktelement eine Kugel 71 angeordnet, die Kräfte von der nicht dargestellten distalen Komponente aufnimmt und auf das eigentliche Sensorelement, das beispielsweise als Drucksensor ausgebildet und piezoresistiv oder kapazitiv arbeitet, überträgt. Alternativ kann das Funktionselement 70 mit einem Dehnmessstreifen beklebt sein, um zu ermitteln, ob und mit welcher Kraft die distale Komponente 30 gegen die Kugel 71 drückt. Grundsätzlich ist es auch möglich, beidseitig Sensoren 70 als Funktionselemente einzubauen, um bei einem Orthesengelenk, das beidseitig ausgehend von einer Ausgangsstellung, verschwenkbar ist, in jeder Belastungsrichtung Sensordaten zu erfassen. Bei einer KAFO oder AFO ist es möglich, darüber einen Bodenkontakt, die Stärke des Bodenkontaktes sowie ein toe-off zu detektieren.

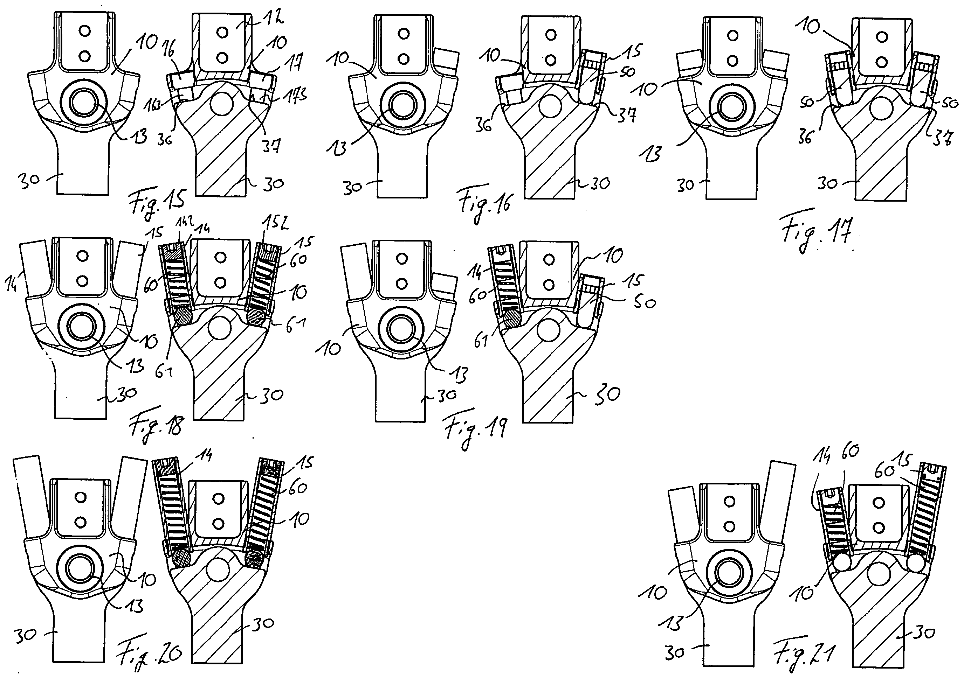

In der Figur 15 ist ein Orthesengelenk in zwei Ansichten mit einem Grundkörper 10, wie er in der Figur 1 dargestellt ist, gezeigt. An dem Grundkörper 10 ist als distale Komponente 30 eine Orthesenschiene schwenkbar gelagert, so dass eine Verschwenkbarkeit um die Lagerstelle 13 gegeben ist. In der rechten Darstellung der Figur 15 ist zu erkennen, dass die distale Komponente 30 beidseitig neben der Lagerstelle 13 Auflagerflächen 36, 37 ausbildet, die den Kanälen 163, 173 der Ausnehmungen 16, 17 zugeordnet sind. Dadurch ist es möglich, Funktionselemente durch Einschrauben oder sonstiges Befestigen in die Ausnehmungen 16, 17 in unmittelbarem Kontakt mit den Auflagerflächen 36, 37 der distalen Komponente 30 zu bringen.

In der Figur 16 ist ein Ausführungsbeispiel dargestellt, bei der nur ein Aufnahmeelement 15 in die Ausnehmung 17 eingeschraubt ist. Als Funktionselement 50 ist ein Anschlag vorgesehen, der über das aus Übersichtsgründen nicht eingezeichnete Innengewinde 150 und ein korrespondierendes Außengewinde an dem Anschlag 50 in der Position veränderlich ist. Dadurch ist es möglich, den maximal erreichbaren Verschwenkwinkel des Grundkörpers 10 relativ zu der distalen Komponente 30 einzustellen.

In der Figur 17 sind in einer Seitenansicht und in einer Schnittdarstellung eine Variante der Erfindung gezeigt, die dem Aufbau der Figur 10 mit zwei eingeschraubten Anschlägen 50 entspricht. Es ist zu erkennen, dass beide Anschläge 50 an den Auflagerflächen 36, 37 des distalen Elementes 30 aufliegen, so dass das Gelenk keine Relativbewegung zulässt.

In der Figur 18 ist die Variante der Figur 12 mit zwei eingeschraubten Federkanälen als Aufnahmeelemente 14, 15 gezeigt. Darüber hinaus sind in den Aufnahmeelementen 14, 15 je eine Feder 60 als Funktionselement angeordnet, die über Kugeln 61 auf die Auflagerflächen 36, 37 der distalen Komponente 30 einwirken. Über einschraubbare Stopfen 142, 152 kann die Vorspannung der Federn 60 eingestellt werden.

Figur 19 zeigt die Variante der Figuren 6 bis 9 im fertig montierten Zustand. Das Aufnahmeelement 14 dient zur Aufnahme einer Feder 60, das gegenüberliegende Aufnahmeelement 15 zur Aufnahme eines Anschlages 50. Dadurch ist es möglich, eine Verdrehung um die Lagerstelle 13 in Richtung auf das Federelement 60 zu ermöglichen, der Anschlag 50 blockiert eine Verschwenkbewegung des Grundkörpers 10 in Uhrzeigerrichtung.

In der Figur 20 sind die Aufnahmeelemente 14, 15 in einer verlängerten Version im Vergleich zu der Figur 18 dargestellt. Hieraus lässt sich erkennen, dass durch den modularen Aufbau unterschiedliche Federn 60 einfach in den Grundkörper 10 einsetzbar und daran fixierbar sind. Dadurch ist es möglich, längere oder stärkere Federn 60 einzubauen, wenn dies seitens der Patienten gewünscht wird.

In der Figur 21 ist eine Kombination der Ausführungsformen der Figuren 18 und 20 gezeigt, das linke Aufnahmeelement 14 ist kürzer als das rechte Aufnahmeelement, in beiden Aufnahmeelementen 14, 15 sind Federn 60 als Funktionselemente angeordnet, so dass es möglich ist, in unterschiedlichen Schwenkrichtungen, ausgehend von der dargestellten Ausgangsstellung, unterschiedliche Federkräfte einer Verschwenkung entgegenwirken zu lassen.

## Patentansprüche

1. Orthesengelenk mit einem Grundkörper (10), der eine Aufnahme (12) für eine proximale Komponente (20) sowie eine Lagerstelle (13) für eine schwenkbar an dem Grundkörper (10) angeordnete distale Komponente (30) und auf die distale Komponente (30) einwirkende Anschläge oder Federelemente aufweist, wobei an dem Grundkörper (10) zumindest ein Aufnahmeelement (14, 15) lösbar befestigt ist, in oder an dem ein mit der distalen Komponente (30) wechselwirkendes Funktionselement (50, 60, 70) angeordnet ist, **dadurch gekennzeichnet, dass** das Aufnahmeelement (14, 15) über die Kontur des Grundkörpers (10) hinaussteht und das Aufnahmeelement (14, 15) und das Funktionselement (50, 60, 70) als eine integrierte Baugruppe ausgebildet sind, so dass sich ein modularer Aufbau ergibt.

2. Orthesengelenk nach Anspruch 1, **dadurch gekennzeichnet, dass** das Aufnahmeelement (14, 15) formschlüssig reversibel an dem Grundkörper (10) festgelegt ist.

3. Orthesengelenk nach Anspruch 1 oder 2, **dadurch gekennzeichnet, dass** das Aufnahmeelement (14, 15) als Hülse mit zumindest einem Gewinde (140, 141, 150, 151) ausgebildet ist.

4. Orthesengelenk nach einem der voranstehenden Ansprüche, **dadurch gekennzeichnet, dass** das Funktionselement (50, 60, 70) als Feder, Federpaket, Dämpfer, Sensor oder Anschlag oder einer Kombination daraus ausgebildet ist.

5. Orthesengelenk nach einem der voranstehenden Ansprüche, **dadurch gekennzeichnet, dass** mehrere Funktionselemente (50, 60, 70) seriell hintereinander angeordnet sind.

6. Orthesengelenk nach einem der voranstehenden Ansprüche, **dadurch gekennzeichnet, dass** das Funktionselement als induktiver Sensor (70) ausgebildet ist, der einen Verlagerungsweg erfasst.

7. Orthesengelenk nach einem der voranstehenden Ansprüche, **dadurch gekennzeichnet, dass** das Aufnahmeelement (14, 15) in einer Ausnehmung (16, 17) in dem Grundkörper (10) eingesetzt ist, in der Formschlusselemente (160, 170) zur Befestigung des Aufnahmeelementes (14, 15) angeordnet sind und dass von der Ausnehmung (16, 17) ein Kanal (163, 173) in Richtung auf die distale Komponente (30) führt.

8. Orthesengelenk nach einem der voranstehenden Ansprüche, **dadurch gekennzeichnet, dass** die Aufnahme (12) als Schienenkasten ausgebildet ist.

9. Orthesengelenk nach einem der voranstehenden Ansprüche, **dadurch gekennzeichnet, dass** die distale Komponente (30) als Schiene, Schienenkasten, Fußbügel oder Fußstütze ausgebildet ist.

10. Orthesengelenk nach einem der voranstehenden Ansprüche, **dadurch gekennzeichnet, dass** Aufnahmeelemente (14,15) beidseitig der Lagerstelle (13) angeordnet sind.

## Claims

1. Orthosis joint having a base body (10) which comprises a receptacle (12) for a proximal component (20), a bearing point (13) for a distal component (30) arranged pivotably on the base body (10), and limit stops or spring elements which act on the distal component (30), wherein at least one receiving element (14, 15), in or on which a functional element (50, 60, 70) interacting with the distal component (30) is arranged, is releasably secured to the base body (10), **characterized in that** the receiving element (14, 15) protrudes beyond the contour of the base body (10) and the receiving element (14, 15) and the functional element (50, 60, 70) are formed as an integrated assembly, resulting in a modular structure.

2. Orthosis joint as claimed in claim 1, **characterized in that** the receiving element (14, 15) is reversibly secured with a form fit to the base body (10).

3. Orthosis joint as claimed in claim 1 or 2, **characterized in that** the receiving element (14, 15) is designed as a sleeve with at least one thread (140, 141, 150, 151).

4. Orthosis joint as claimed in one of the preceding claims, **characterized in that** the functional element (50, 60, 70) is designed as a spring, spring assembly, damper, sensor or limit stop, or a combination of these.

5. Orthosis joint as claimed in one of the preceding claims, **characterized in that** several functional elements (50, 60, 70) are arranged one behind another in series.

6. Orthosis joint as claimed in one of the preceding claims, **characterized in that** the functional element is designed as an inductive sensor (70), which detects a displacement path.

7. Orthosis joint as claimed in one of the preceding claims, **characterized in that** the receiving element (14, 15) is inserted in a recess (16, 17) in the base body (10), in which recess (16, 17) form-fit elements (160, 170) are arranged for securing the receiving element (14, 15), and **in that** a channel (163, 173) leads from the recess (16, 17) in the direction of the distal component (30).

8. Orthosis joint as claimed in one of the preceding claims, **characterized in that** the receptacle (12) is designed as a rail box.

9. Orthosis joint as claimed in one of the preceding claims, **characterized in that** the distal component (30) is designed as a rail, rail box, foot stirrup or foot support.

10. Orthosis joint as claimed in one of the preceding claims, **characterized in that** receiving elements (14, 15) are arranged on both sides of the bearing point (13).

## Revendications

1. Articulation orthétique comprenant un corps de base (10), qui comporte un récepteur (12) pour un composant proximal (20) ainsi qu'un emplacement de montage (13) pour un composant distal (30) agencé avec faculté de pivotement sur le corps de base (10), et des butées ou des éléments à ressort agissant sur le composant distal (30), dans laquelle au moins un élément récepteur (14,15) est fixé de façon détachable sur le corps de base (10), élément récepteur dans lequel ou sur lequel est agencé un élément fonctionnel (50, 60, 70) qui coopère avec le composant distal (30), **caractérisé en ce que** l'élément récepteur (14, 15) dépasse au-delà du contour du corps de base (10), et l'élément récepteur (14, 15) et l'élément fonctionnel (50, 60, 70) sont réalisés sous la forme d'un groupe structurel intégré, de sorte qu'il en résulte une structure modulaire.

2. Articulation orthétique selon la revendication 1, **caractérisée en ce que** l'élément récepteur (14, 15) est immobilisé par coopération de formes et de manière réversible sur le corps de base (10).

3. Articulation orthétique selon la revendication 1 ou 2, **caractérisée en ce que** l'élément récepteur (14,15) est réalisé comme une douille avec au moins un pas de vis (140, 141, 150, 151).

4. Articulation orthétique selon l'une des revendications précédentes, **caractérisée en ce que** l'élément fonctionnel (50, 60, 70) est réalisé sous forme de ressort, d'empilage-ressort, d'amortisseur, de capteur ou de butée, ou d'une combinaison de ceux-ci.

5. Articulation orthétique selon l'une des revendications précédentes, **caractérisée en ce que** plusieurs éléments fonctionnels (50, 60, 70) sont agencés en série les uns derrière les autres.

6. Articulation orthétique selon l'une des revendications précédentes, **caractérisée en ce que** l'élément fonctionnel est réalisé sous forme de capteur inductif (70) qui détecte un trajet de déplacement.

7. Articulation orthétique selon l'une des revendications précédentes, **caractérisée en ce que** l'élément récepteur (14, 15) est mis en place dans un évidement (16, 17) dans le corps de base (10), dans lequel sont agencés des éléments à coopération de formes (160, 70) destinés à la fixation de l'élément récepteur (14, 15), et **en ce qu'**un canal (163, 173) mène depuis l'évidement (16, 17) en direction du composant distal (30).

8. Articulation orthétique selon l'une des revendications précédentes, **caractérisée en ce que** le logement (12) est réalisé sous forme de casier à glissières.

9. Articulation orthétique selon l'une des revendications précédentes, **caractérisée en ce que** le composant distal (30) est réalisé sous forme de tringle, de casier à glissières, d'étrier pour pied ou de soutien pour pied.

10. Articulation orthétique selon l'une des revendications précédentes, **caractérisée en ce que** des éléments récepteurs (14, 15) sont agencés sur les deux côtés de l'emplacement de montage (13).
